# FASCICULE DE BREVET EUROPEEN

(11) **EP 1 192 123 B1**
(45) Date de publication et mention de la délivrance du brevet: **21.04.2004**
(21) Numéro de dépôt: 01907644.7
(22) Date de dépôt: 19.01.2001
(51) Int. Cl.: C07C 219/08, C07C 213/08, C08F 20/34

(54) **NOUVEAUX MONOMERES A GROUPES AMINO QUATERNAIRES, LEUR PROCEDE DE FABRICATION, ET LES NOUVEAUX (CO)POLYMERES OBTENUS A PARTIR DE CES NOUVEAUX MONOMERES**
MONOMERE MIT QUATERNÄREN AMINOGRUPPEN, VERFAHREN ZU DEREN HERSTELLUNG, (CO)POLYMERE AUS DIESEN MONOMEREN
NOVEL MONOMERS WITH QUATERNARY AMINO GROUPS, METHOD FOR MAKING SAME, AND NOVEL (CO)POLYMERS OBTAINED FROM SAID NOVEL MONOMERS

(30) Priorité: 24.01.2000 FR 0000836
(43) Date de publication de la demande: 03.04.2002
(73) Titulaire: Atofina, 92800 Puteaux (FR)
(72) Inventeur: RIONDEL, Alain, F-57600 Forbach (FR); CHAPLINSKI, Vladimir, F-57500 Saint Avold (FR); TEMBOU N'ZUDIE, Denis, F-27470 Serquigny (FR)
(74) Mandataire: Chaillot, Geneviève
(86) Numéro de dépôt international: PCT/FR2001/000180
(87) Numéro de publication internationale: WO 2001/055088

(56) Documents cités:
- EP-A- 0 250 325
- CZ-A- 250 962
- FR-A- 1 529 000
- V.N. USHAKOVA ET AL.: "Radiation-induced copolymerization of N-Vinylpyrrolidone with quaternary ammonium salts of 1,3-bis(dimethylamino)isopropyl methacrylate" RUSSIAN JOURNAL OF APPLIED CHEMISTRY., vol. 69, no. 2, 1996, pages 270-273, XP002150046 CONSULTANTS BUREAU., US ISSN: 1070-4272
- SOLOVSKII, M. V. ET AL: "Synthesis and antimcrobial properties of mono- and polymeric quaternary ammonium salts containing aminoalkyl esters of methacrylic acid" KHIM.-FARM. ZH. (1974), 8(6), 20-4, 1974, XP000952593
- KORSHUNOV, M. A. ET AL: "Esters of.alpha.,.beta.-unsaturated acids with functional groups in the alkoxy radical. VII. Acrylates and methacrylates of monohydric polyamino alcohols" ZH. ORG. KHIM. (1969), 5(11), 1947-52, 1969, XP000952682

## Description

La présente invention porte sur de nouveaux monomères à groupes amino quaternaires, sur leur procédé de fabrication, et sur les nouveaux (co)polymères obtenus à partir de ces nouveaux monomères.

Les composé du type de ceux de formule : 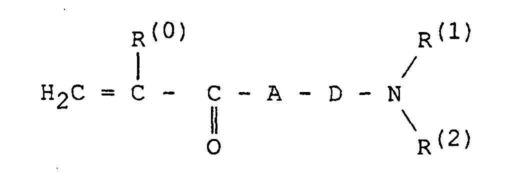 dans laquelle :
- R⁽⁰⁾ représente H ou: CH₃ ;
- A représente -O- ou -NH- ;
- D représente une chaîne alkylène linéaire ou ramifiée en C₁-C₆ ;
- R⁽¹⁾ et R⁽²⁾, identiques ou différents, représentent chacun indépendamment H ou alkyle en C₁₋₅ ;
sont bien connus dans la littérature.

Des composés importants de cette famille sont l'acrylate de N,N-diméthylamino éthyle (ADAME) et le méthacrylate de N,N-diméthylamino éthyle (MADAME) : 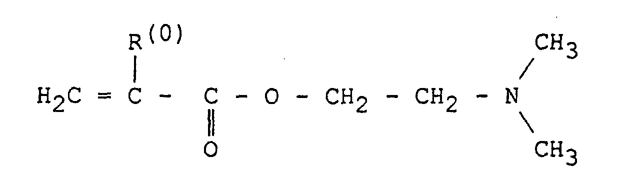 avec R⁽⁰⁾ = H ou CH₃.

Une très nombreuse littérature-brevets décrit la fabrication de solutions aqueuses de sels d'ammonium quaternaire, à partir de l'ADAME et du MADAME, ces sels, pour les plus représentatifs d'entre eux, pouvant être représentés par la formule : 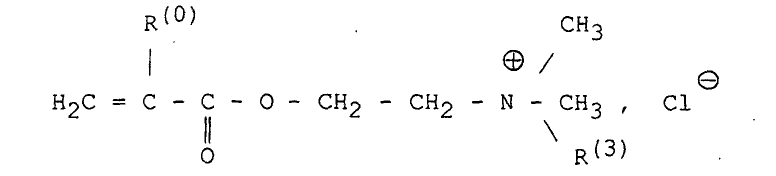 avec R⁽⁰⁾ = H ou CH₃ et R⁽³⁾ = CH₃ ou benzyle, ces sels pouvant être désignés par l'abréviation (M)ADAMQUAT MC ou (M)ADAMQUAT BZ suivant que R⁽³⁾ représente CH₃ ou benzyle.

Cette réaction est une quaternisation, en présence d'eau, du composé de départ avec un agent quaternisant R⁽³⁾ - Cl. La demande de brevet européen EP-A-0 250 325 décrit un procédé de préparation de solutions aqueuses de tels sels d'ammonium quaternaire insaturés.

Les solutions aqueuses de sels quaternaires ainsi obtenues servent notamment à préparer des polymères destinés à servir de floculants cationiques dans le traitement des eaux.

Par le brevet CZ-A-250 962, on connaît les composés de formule : 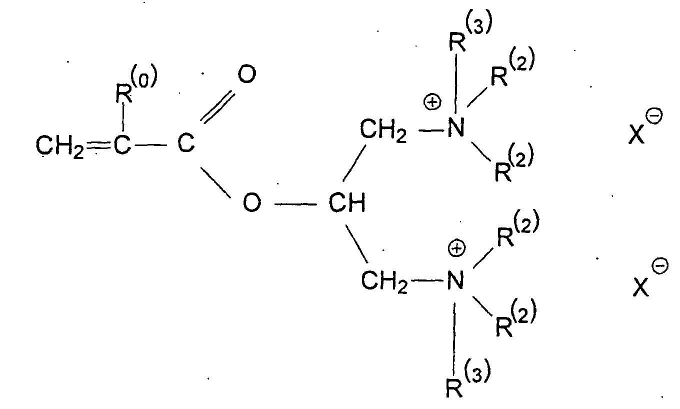 dans laquelle :
- R⁽⁰⁾ représente H ou -CH₃ ;
- R⁽²⁾ représente -CH₃, -C₂H₅, -C₃H₇ ou -C₄H₉ ;
- R⁽³⁾ représente -CH₃, -C₂H₅, -C₃H₇, -C₄H₉, C₆H₅ ou -CH₂C₆H₅ ; et
- X^{⊖} représente Cl^{⊖} ou Br^{⊖}.

Au cours de travaux de recherche et de développement, la Société déposante a découvert de nouveaux monomères, lesquels font l'objet de la présente demande, ainsi que leur procédé de fabrication et les homo- ou copolymères comportant des motifs dérivés de ces nouveaux monomères.

Des dispersions aqueuses salines ou sans sel réalisées à partir de ces nouveaux monomères et apportant une solution à des problèmes techniques posés à l'homme du métier font l'objet de trois demandes de brevets français déposées ce jour au nom de la Société déposante.

La présente invention a donc d'abord pour objet les composés de formule (I) : 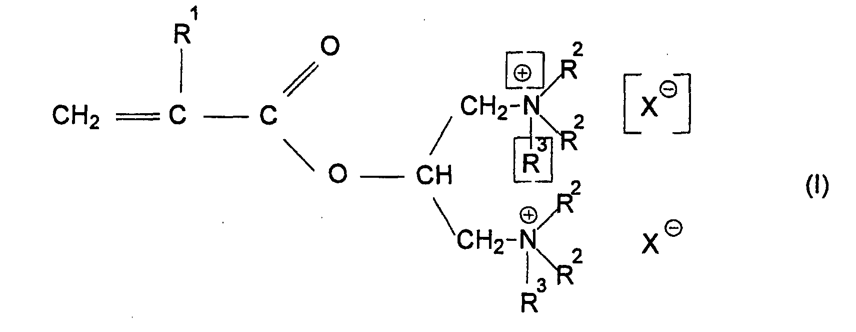 dans laquelle :
- R¹ représente H ou -CH₃ ;
- R² représente -CH₃ ; -C₂H₅ ; -C₃H₇ ou -C₄H₉ ; et
- le composé (I) est facultativement quaternisé sur l'un des azotes, ce qui est symbolisé par le fait que les R³, X^{⊖} et ^{⊕} associés à cet azote sont entre crochets ;
- lorsque le composé (I) est quaternisé sur un seul azote, R³ et X^{⊖} ont les significations suivantes:
   (1) R³ représente -CH₃ ou -CH₂C₆H₅ ; et X^{⊖} représente Cl^{⊖} ou CH₃OSO₃^{⊖} ; ou
   (2) R³ représente un groupement alkyle en C₁-C₁₂ ; et X^{⊖} représente Br^{⊖} ou I^{⊖} ;
- lorsque le composé (I) est quaternisé sur les deux azotes, les deux X^{⊖} peuvent être identiques ou différents et les deux R³ peuvent être identiques, auquel cas :
   (3) R³ représente un groupement alkyle en C₅-C₁₂ ; et X^{⊖} représente CH₃OSO₃^{⊖}, Br^{⊖} ou I^{⊖} ;
   ou différents, auquel cas :
   (4) l'un des R³ représente -CH₃ ou -CH₂C₆H₅ ; et X^{⊖} représente Cl^{⊖} ou CH₃OSO₃^{⊖} ; et
      l'autre représente un groupe alkyle en C₅-C₁₂ ; et X^{⊖} représente Br^{⊖} ou le ;
et les mélanges de ces composés.

A titre d'exemple de composé (I), on peut citer le composé de formule (Ia) : 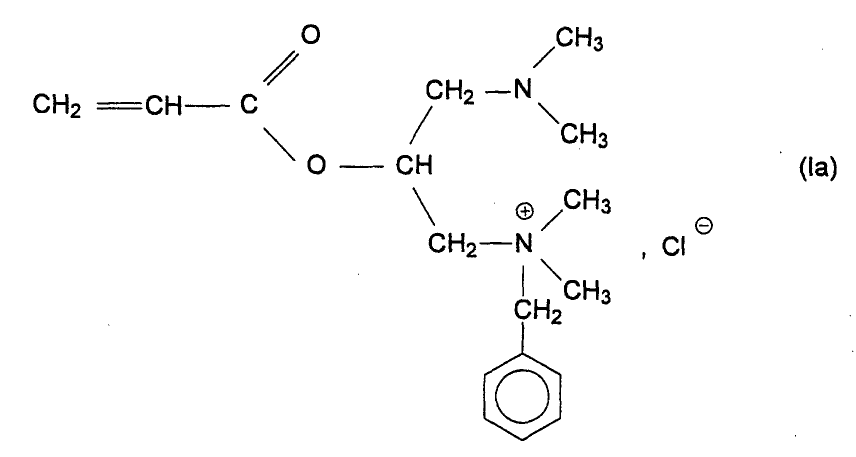 que l'on pourra désigner par l'abréviation S-ADAMQUAT BZ.

Les composés de l'invention peuvent avantageusement se présenter en solution aqueuse.

La présente invention a également pour objet un procédé de fabrication des composés de formule (I) tels que définis ci-dessus, caractérisé par le fait que l'on introduit, à une température de 35 à 80°C, dans une solution dans un solvant organique ou un mélange de solvants organiques d'un composé de formule (II) : 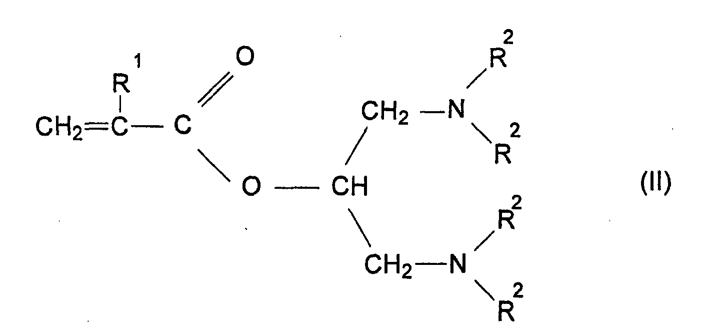 dans laquelle R¹ et R² sont tels que définis ci-dessus, un agent quaternisant de formule (III) :

R³ - X^{⊖} (III)

dans laquelle :
- R³ et X^{⊖} ont les significations (1) et (2) indiquées ci-dessus, auquel cas le rapport molaire agent quaternisant (III) / composé (II) est compris entre 0,9 et 1,5, et l'on obtient un composé (I) quaternisé sur un seul azote ; ou
- R³ et X^{⊖} ont la signification (3) indiquée ci-dessus ou deux agents quaternisants différents sont introduits, les R³ et X^{⊖} de ces deux agents quaternisants ayant la signification (4) indiquée ci-dessus, auquel cas le rapport molaire agent(s) quaternisant(s) (III) / composé (II) est compris entre 0,9 et 1,5, et l'on obtient un composé (I) quaternisé sur les deux azotes,
puis qu'on laisse se dérouler la réaction à ladite température jusqu'à disparition complète ou sensiblement complète du ou des composés (III), et qu'on ajoute de l'eau, puis qu'on sépare une solution aqueuse de composé (I), et qu'on élimine l'eau le cas échéant.

Comme solvant(s) organique(s), on utilise, par exemple, le chloroforme, le dichlorométhane, le dichloroéthane et leurs mélanges.

La réaction n'est généralement pas conduite sous pression, excepté si au moins un agent quaternisant (III) est à l'état gazeux.

On introduit le ou les agents quaternisants (III) dans la solution du composé (II) généralement en l'espace de 0,5 - 2 heures, et, après l'introduction de la totalité du ou des agents quaternisants, on conduit la réaction des composés (II) et (III) généralement pendant un laps de temps de 10 à 40 heures.

Après la séparation de la solution aqueuse de composé (I), on préfère débarrasser la solution aqueuse obtenue de toute trace de solvant organique par stripping à l'air sous pression réduite.

Le procédé précité conduit à une solution aqueuse ayant une concentration en composé (I) qui est, de préférence, de 65 à 75% en poids.

Conformément à une caractéristique particulière du procédé ci-dessus, celui-ci est conduit en présence d'au moins un stabilisant choisi notamment parmi l'hydroquinone, l'éther méthylique de l'hydroquinone et le 3,5-ditert.-butyl-4-hydroxytoluène et les mélanges de ces stabilisants, la teneur en agent(s) stabilisant(s) étant notamment de 400 à 2 000 ppm par rapport à la solution aqueuse de composé (I) final.

On peut préparer le composé (II) en faisant réagir un composé de formule (IV) : 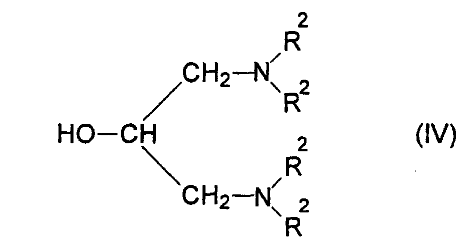 dans laquelle R² est tel que défini ci-dessus, avec l'anhydride (méth)acrylique en présence de triéthylamine, avec un rapport molaire anhydride (méth)acrylique/composé (IV) de 0,5 à 2, à une température de 20 à 100°C, en particulier de 30 à 60°C, pendant un laps de temps de 2 à 10 heures,
en présence d'au moins un stabilisant, tel que la phénothiazine, l'éther méthylique de l' hydroquinone, le 3, 5-ditert.-butyl-4-hydroxytoluène et l'hydroquinone, et les mélanges de ces stabilisants, à raison de 200 à 3000 ppm par rapport à la charge.

Dans la réaction avec l'anhydride (méth) acrylique, la triéthylamine sert à catalyser la réaction et à piéger l'acide (méth)acrylique formé sous forme de sel. Elle est utilisée généralement à raison de 1 à 2 équivalents molaires par rapport à l'anhydride (méth)acrylique.

La présente invention a également pour objet des homopolymères ou copolymères comportant des motifs d'au moins un monomère de formule (I) tel que défini ci-dessus.

Les copolymères à base des monomères (I) incluant le monomère (Ia) peuvent être des polymères hydrosolubles ou hydrophobes ayant une présentation sous forme de dispersion aqueuse, latex, solution aqueuse, émulsion inverse ou de poudre. Ils sont préparés par copolymérisation radicalaire selon divers procédés de synthèse tels que les procédés de polymérisation en dispersion, solution, émulsion directe, émulsion inverse et suspension inverse.

Les Exemples qui vont suivre, donnés à titre indicatif, permettent de mieux comprendre l'invention. Dans ces exemples, les parties et pourcentages indiqués sont en poids sauf indication contraire.

Dans ces exemples, les abréviations suivantes ont été utilisées :
- S-ADAME :: composé de formule : 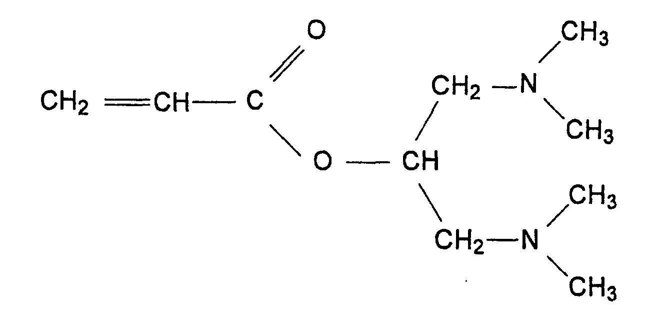
- EMHQ :: éther méthylique de l'hydroquinone.

### EXEMPLE 1 : Synthèse du S-ADAME

Dans un réacteur en verre de 1 litre, on charge :
- 292 g de 1,3-bis-diméthylamino-2 propanol ;
- 242 g de triéthylamine ; et
- 0,373 g de phénothiazine en tant que stabilisant.

Dans ce mélange agité, sous bullage d'air, à température ambiante, on ajoute, en 1 heure, 226 g d'anhydride acrylique. La température augmente pour atteindre 50°C. Après 2 heures supplémentaires de réaction, le mélange est refroidi et on ajoute 50 ml d'eau. Après décantation, on obtient une phase organique supérieure de 450 g, laquelle est distillée sous pression réduite pour isoler 250 g du composé de l'intitulé (pureté GC ≥ 99%).

### EXEMPLE 2 : Quaternisation du S-ADAME en S-ADAMOUAT BZ

Dans un réacteur en verre de 250 ml, on charge 44,2 g du S-ADAME obtenu au point (a) stabilisé avec 1500 ppm d'éther méthylique de l'hydroquinone et 150 g de CHCl₃. Le mélange sous agitation et sous bullage d'air est porté à 50°C. On ajoute en 1 heure, 28 g de chlorure de benzyle. Après 25 heures de réaction, l'acrylate de départ a disparu et l'on ajoute 33 g d'eau. On décante une phase supérieure qui est débarrassée des traces de CHCl₃ par stripping à l'air à 45°C sous pression réduite (P = 1,33 x 10⁴ Pa) (100 mm Hg)). On obtient ainsi 70 g de solution aqueuse contenant 65% de monomère cationique quaternaire ayant la structure attendue, déterminée par RMN ¹³C. Ce monomère est appelé S-ADAMQUAT BZ.

## Revendications

1. Composés de formule (I) : 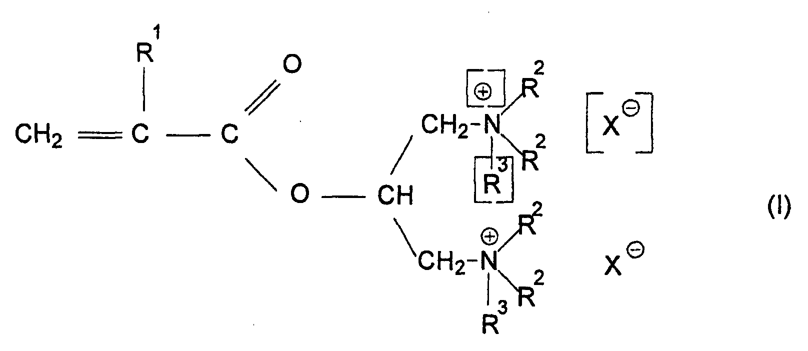 dans laquelle :
- R¹ représente H ou -CH₃ ;
- R² représente -CH₃ ; -C₂H₅ ; -C₃H₇ ou -C₄H₉ ; et
- le composé (I) est facultativement quaternisé sur l'un des azotes, ce qui est symbolisé par le fait que les R³, X^{⊖} et ^{⊕} associés à cet azote sont entre crochets ;
- lorsque le composé (I) est quaternisé sur un seul azote, R³ et X^{⊖} ont les significations suivantes:
(1) R³ représente -CH₃ ou -CH₂C₆H₅ ; et X^{⊖} représente Cl^{⊖} ou CH₃OSO₃^{⊖} ; ou
(2) R³ représente un groupement alkyle en C₁-C₁₂ ; et X^{⊖} représente Br^{⊖} ou I^{⊖} ;
- lorsque le composé (I) est quaternisé sur les deux azotes, les deux X^{⊖} peuvent être identiques ou différents et les deux R³ peuvent être identiques, auquel cas :
(3) R³ représente un groupement alkyle en C₅-C₁₂ ; et X^{⊖} représente CH₃OSO₃^{⊖}, Br^{⊖} ou I^{⊖} ;
ou différents, auquel cas :
(4) l'un des R³ représente -CH₃ ou -CH₂C₆H₅ ; et X^{⊖} représente Cl^{⊖} ou CH₃OSO₃^{⊖} ; et
l'autre représente un groupe alkyle en C₅-C₁₂ ; et X^{⊖} représente Br^{⊖} ou I^{⊖} ;
et les mélanges de ces composés.

2. Composé selon la revendication 1, **caractérisé par le fait qu'**il est représenté par la formule (Ia) :

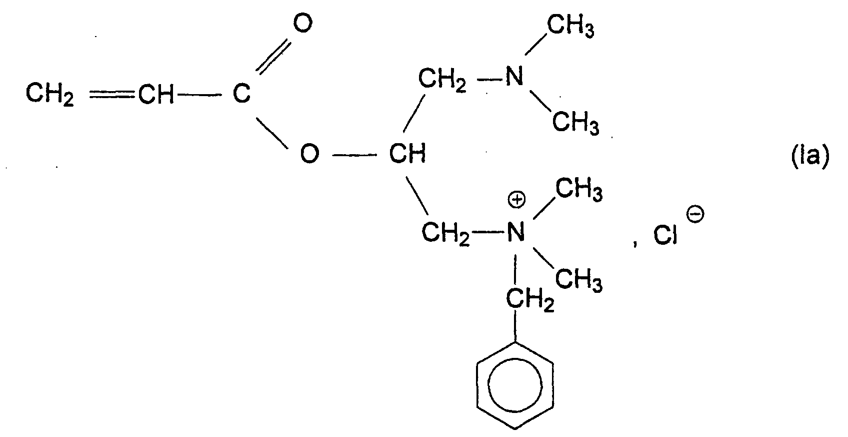

3. Composés selon l'une des revendications 1 et 2, **caractérisés par le fait qu'**ils se présentent en solution aqueuse.

4. Procédé de fabrication des composés tels que définis à l'une des revendications 1 à 3, **caractérisé par le fait que** l'on introduit, à une température de 35 à 80°C, dans une solution dans un solvant organique ou un mélange de solvants organiques d'un composé de formule (II) : 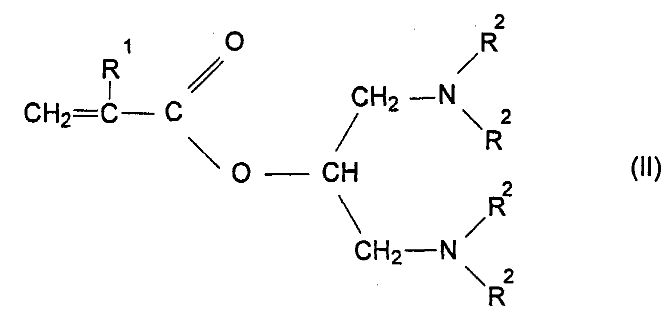 dans laquelle R¹ et R² sont tels que définis à la revendication 1,
un agent quaternisant de formule (III) :
R³ - X^{⊖} (III)
dans laquelle :
- R³ et X ^{⊖} ont les significations (1) et (2) indiquées à la revendication 1, auquel cas le rapport molaire agent quaternisant (III) / composé (II) est compris entre 0,9 et 1,5, et l'on obtient un composé (I) quaternisé sur un seul azote ; ou
- R³ et X^{⊖} ont la signification (3) indiquée à la revendication 1 ou deux agents quaternisants différents sont introduits, les R³ et X ^{⊖} de ces deux agents quaternisants ayant la signification (4) indiquée à la revendication 1, auquel cas le rapport molaire agent(s) quaternisant(s) (III) / composé (II) est compris entre 0,9 et 1,5, et l'on obtient un composé (I) quaternisé sur les deux azotes,
puis qu'on laisse se dérouler la réaction à ladite température jusqu'à disparition complète ou sensiblement complète du ou des composés (III), et qu'on ajoute de l'eau, puis qu'on sépare une solution aqueuse de composé (I), et qu'on élimine l'eau le cas échéant.

5. Procédé selon la revendication 4, **caractérisé par le fait que**, comme solvant organique, on utilise au moins l'un parmi le chloroforme, le dichlorométhane et le dichloroéthane.

6. Procédé selon l'une des revendications 4 et 5, **caractérisé par le fait qu'**il est conduit sous pression si l'agent quaternisant ou au moins un agent quaternisant (III) est à l'état gazeux.

7. Procédé selon l'une des revendication 4 à 6, **caractérisé par le fait que** l'on introduit l'agent quaternisant ou les agents quaternisants (III) dans la solution du composé (II) en l'espace de 0,5 - 2 heures.

8. Procédé selon l'une des revendication 4 à 7, **caractérisé par le fait qu'**après l'introduction de la totalité de l'agent quaternisant ou des agents quaternisants, on conduit la réaction des composés (II) et (III) pendant un laps de temps de 10 à 40 heures.

9. Procédé selon l'une des revendication 4 à 8, **caractérisé par le fait que** l'on débarrasse la solution aqueuse obtenue de toute trace de solvant organique par stripping à l'air sous pression réduite.

10. Procédé selon l'une des revendication 4 à 9, **caractérisé par le fait qu'**il conduit à une solution aqueuse ayant une concentration en composé (I) de 65 à 75% en poids.

11. Procédé selon l'une des revendication 4 à 10, **caractérisé par le fait qu'**il est conduit en présence d'au moins un stabilisant choisi notamment parmi l'hydroquinone, l'éther méthylique de l'hydroquinone et le 3,5-ditert.-butyl-4-hydroxytoluène et les mélanges de ces stabilisants, la teneur en agent(s) stabilisant(s) étant notamment de 400 à 2000 ppm par rapport à la solution aqueuse de composé (I) final.

12. Procédé selon l'une des revendications 4 à 11, **caractérisé par le fait que** l'on prépare le composé (II) en faisant réagir un composé de formule (IV): 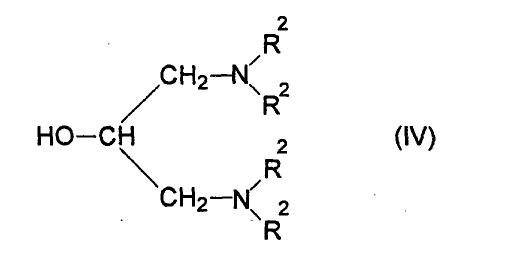 dans laquelle R² est tel que défini ci-dessus,
avec l'anhydride (méth)acrylique en présence de triéthylamine, avec un rapport molaire anhydride (méth)acrylique/composé (IV) de 0,5 à 2, à une température de 20 à 100°C, en particulier de 30 à 60°C, pendant un laps de temps de 2 à 10 heures, en présence d'au moins un stabilisant, tel que la phénothiazine, l'éther méthylique de l'hydroquinone, le 3,5-ditert.-butyl-4-hydroxytoluène et l'hydroquinone, et les mélanges de ces stabilisants, à raison de 200 à 3000 ppm par rapport à la charge.

13. Homopolymères ou copolymères comportant des motifs d'au moins un monomère de formule (I) tel que défini à l'une des revendications 1 à 3.

## Claims

1. Compounds having formula (I): 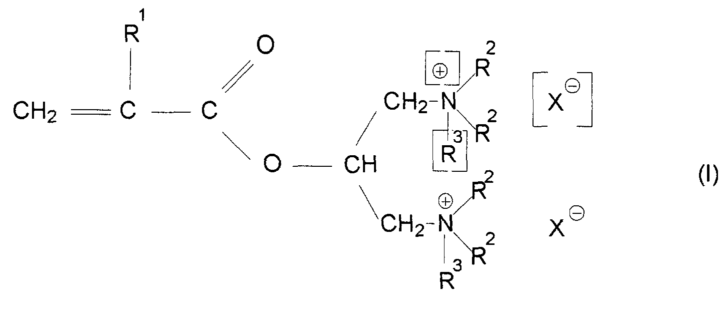 in which :
- R¹ represents H or -CH₃;
- R² represents -CH₃; -C₂H₅; -C₃H₇ or -C₄H₉; and
- compound (I) is optionally quaternised on one of the nitrogens, which is symbolised by the fact that the R³, X ^{⊖} and ⊕ associated with this nitrogen are shown between brackets;
- if compound (I) is quaternised on a single nitrogen, R³ and X ^{⊖} have the following meanings :
(1) R³ represents -CH₃ or -CH₂C₆H₅ ; and X ^{⊖} represents Cl^{⊖} or CH₃OSO₃^{⊖}; or
(2) R³ represents a C₁-C₁₂-alkyl group ; and X^{⊖} represents Br^{⊖} or I^{⊖} ;
- if compound (I) is quaternised on both nitrogens, the two X^{⊖} may be the same or different and the two R³ may be the same, in which case:
(3) R³ represents a C₅-C₁₂-alkyl ; and X^{⊖} represents CH₃OSO₃^{⊖}, Br^{⊖} or I^{⊖} ;
or different, in which case:
(4) one of the R³ represents -CH₃ or -CH₂C₆H₅ ; and X^{⊖} represents Cl^{⊖} or CH₃OSO₃ ^{⊖} ; and the other represents a C₅-C₁₂-alkyl group ; and X^{⊖} represents Br^{⊖} or I^{⊖} ;
and mixtures of these compounds.

2. Compound as claimed in claim 1, **characterised in that** it is represented by the formula (Ia) :

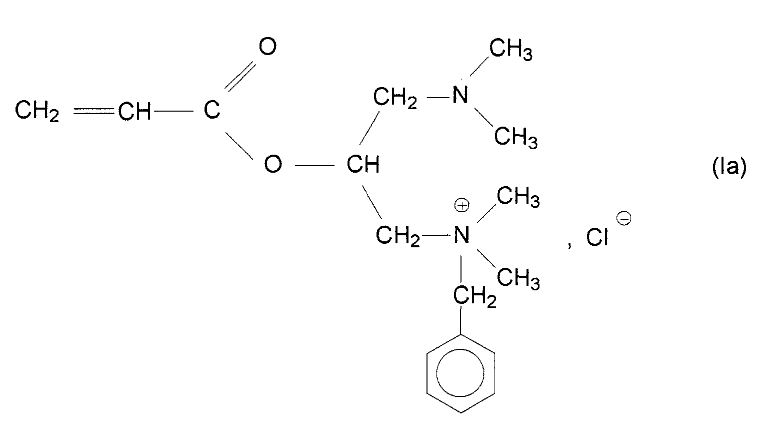

3. Compounds as claimed in one of claims 1 and 2, **characterised in that** they are in the form of an aqueous solution.

4. Method of manufacturing compounds as defined in one of claims 1 to 3, **characterised in that** into a solution in an organic solvent or a mixture of organic solvents, of a compound of formula (II): 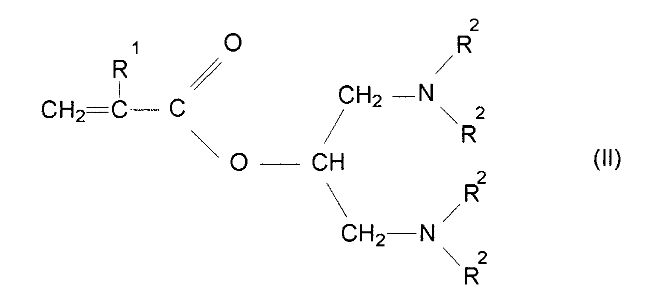 in which R¹ and R² are as defined in claim 1,
is introduced, at a temperature of 35 to 80°C, a quaternising agent of formula (III):
R³ - X^{⊖} (III)
in which:
- R³ and X^{⊖} have the meanings (1) and (2) specified in claim 1, in which case the molar ratio of quaternising agent (III) / compound (II) is in a range of between 0.9 and 1.5 and a compound(I) is obtained which is quaternised on a single nitrogen ; or
- R³ and X^{⊖} have meaning (3) specified in claim 1 or two different quaternising agents are used, the R³ and X^{⊖} of these two quaternising agents having meaning (4) specified in claim 1, in whichcase the molar ratio of quaternising agent(s) (III) / compound (II) is in a range of between 0.9 and 1.5 and a compound (I) is obtained which is quaternised on both nitrogens,
then the reaction is allowed to continue at said temperature until compound(s) (III) has/have disappeared completely or substantially completely, after which water is added and then an aqueous solution of compound (I) is separated and the water removed as necessary.

5. Method as claimed in claim 4, **characterised in that** the organic solvent used is at least one selected from chloroform, dichloromethane and dichloroethane.

6. Method as claimed in one of claims 4 and 5, **characterised in that** it is conducted under pressure if the quaternising agent or at least one quaternising agent (III) is in the gaseous state.

7. Method as claimed in one of claims 4 to 6, **characterised in that** the quaternising agent or quaternising agents (III) is/are introduced into the solution of compound (II) over a period of 0.5 - 2 hours.

8. Method as claimed in one of claims 4 to 7, **characterised in that** once all of the quaternising agent or quaternising agents has/have been introduced, the reaction between compounds (II) and (III) is conducted for a period of between 10 and40 hours.

9. Method as claimed in one of claims 4 to 8, **characterised in that** all traces of organic solvent are removed from the resultant aqueous solution by stripping in air at a reduced pressure.

10. Method as claimed in one of claims 4 to 9, **characterised in that** it produces an aqueous solution with a concentration of 65 to 75% by weight of compound (I) .

11. Method as claimed in one of claims 4 to 10, **characterised in that** it is conducted inthe presence of at least one stabiliser chosen in particular from hydroquinone, hydroquinone methyl ether and 3,5-ditert.-butyl-4-hydroxytoluene and mixtures of these stabilisers, the content of stabilising agent(s) being in particular from 400 to 2000 ppm relative to the aqueous solution of the final compound (I).

12. Method as claimed in one of claims 4 to 11, **characterised in that** compound (II) is prepared by causing a compound of formula (IV): 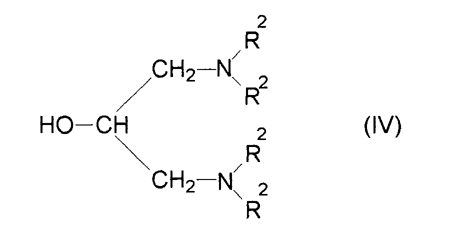 in which R² is as defined above,
to react with (meth)acrylic anhydride in the presence of triethylamine, with a molar ratio of (meth)acrylic anhydride / compound (IV) of 0.5 to 2, at a temperature of 20 to 100°C, in particular 30 to 60°C, for a period of 2 to 10 hours in the presence of at least onestabiliser such as phenothiazine, hydroquinone methyl ether, 3,5-ditert.-butyl-4-hydroxytoluene and hydroquinone and mixtures of these stabilisers in a ratio of 200 to 3000 ppm relative to the charge.

13. Homopolymers or copolymers containing units of at least one monomer of formula (I) as defined in one of claims 1 to 3.

## Patentansprüche

1. Verbindungen der Formel (I): 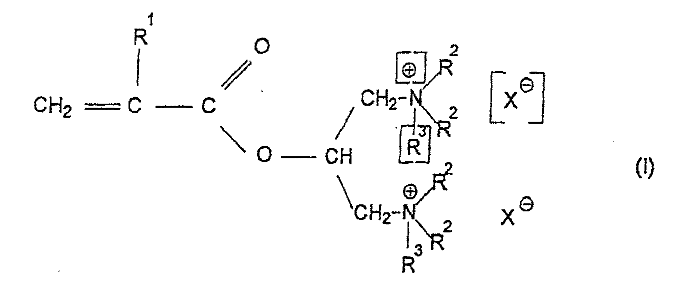 worin:
R¹ H oder -CH₃ repräsentiert;
R²-CH₃; -C₂H₅; -C₃H₇ oder -C₄H₉ repräsentiert; und
die Verbindung (I) optional an einem der Stickstoffatome quartemisiert ist, welches dadurch symbolisiert ist, dass sich R³, X^{⊖} und ^{⊕} in Verbindung mit dem Stickstoffatom in eckigen Klammern befinden;
wenn die Verbindung (I) an einem einzigen Stickstoffatom quartemisiert ist, R³ und X^{⊖} die folgenden Bedeutungen besitzen:
(1) R³ repräsentiert -CH₃ oder -CH₂C₆H₅ ; und X^{⊖} repräsentiert Cl^{⊖} oder CH₃OSO₃^{⊖}; oder
(2) R³ repräsentiert eine C₁-C₁₂alkylgruppe; und X^{⊖} repräsentiert Br^{⊖} oder I^{⊖};
wenn die Verbindung (I) an beiden Stickstoffatomen quartemisiert ist, können beide X^{⊖} identisch oder verschieden sein und die beiden R³ können identisch sein in dem Fall, dass
(3) R³ eine C₅-C₁₂alkylgruppe repräsentiert; und X^{⊖} repräsentiert CH₃OSO₃^{⊖}; Br^{⊖} oder I^{⊖};
oder unterschiedlich, in welchem Fall:
(4) eine der R³ -CH₃ oder-CH₂C₆H₅ repräsentiert; und X^{⊖} repräsentiert Cl^{⊖} oder CH₃OSO₃^{⊖}; und die andere repräsentiert eine C₅-C₁₂alkylgruppe; und X^{⊖} repräsentiert Br^{⊖} oder I^{⊖};
und Mischungen dieser Verbindungen.

2. Verbindung gemäss Anspruch 1, **dadurch gekennzeichnet, dass** diese durch die Formel (Ia) wiedergegeben wird:

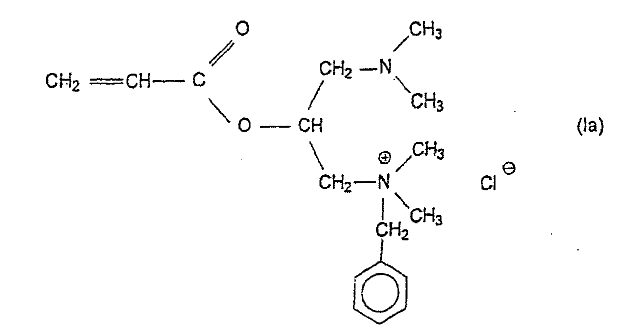

3. Verbindungen gemäss einem der Ansprüche 1 oder 2, **dadurch gekennzeichnet, dass** sie in wässriger Lösung vorliegen.

4. Verfahren zur Herstellung von Verbindungen gemäss einem der Ansprüche 1 bis 3, **dadurch gekennzeichnet, dass** eine Verbindung der Formel (II): 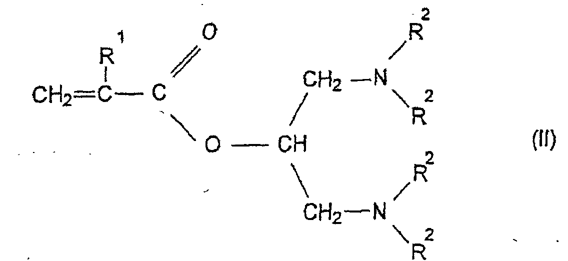 worin R¹ und R² wie in Anspruch 1 definiert sind,
bei einer Temperatur von 35 bis 80 °C in eine Lösung eines organischen Lösungsmittels oder eine Mischung organischer Lösungsmittel eingebracht wird,
ein Quarternisierungsmittel der Formel (III):
R³-X^{⊖} (III)
worin:
R³ und X^{⊖} die in Anspruch 1 angegebenen Bedeutungen (1) und (2) besitzen, wobei das Molverhältnis von Quarternisierungsmittel (III)/Verbindung (II) zwischen 0,9 und 1,5 liegt, und man eine an einem einzigen Stickstoffatom quarternisierte Verbindung (I) erhält; oder
R³ und X^{⊖} die in Anspruch 1 angegebene Bedeutung (3) besitzen oder es werden zwei verschiedene Quarternisierungsmittel eingesetzt, wobei die beiden R³ und
X^{⊖} der beiden Quarternisierungsmittel die in Anspruch 1 angegebene Bedeutung (4) besitzen, in welchem Fall das Molverhältnis der (des) Quarternisierungsmittel(s) (III)/Verbindung (II) zwischen 0,9 und 1,5 liegt, und man eine an beiden Stickstoffatomen quarternisierte Verbindung (I) erhält,
wobei man nachfolgend die Reaktion bei der angegebenen Temperatur ablaufen lässt, bis zum vollständigen Verschwinden oder spürbar vollständigen Verschwinden der Verbindungen (III), und man Wasser hinzufügt, woraufhin sich eine wässrige Lösung der Verbindung (I) bildet und man im vorliegenden Fall das Wasser entfernt.

5. Verfahren gemäß Anspruch 4, **dadurch gekennzeichnet, dass** als organisches Lösungsmittel wenigstens eines von Chloroform, Dichlormethan und Dichlorethan verwendet wird.

6. Verfahren gemäss einem der Ansprüche 4 und 5, **dadurch gekennzeichnet, dass** das Verfahren unter Druck durchgeführt wird, wenn das Quarternisierungsmittel oder wenigstens ein Quarternisierungsmittel (III) in gasförmigem Zustand vorliegt.

7. Verfahren gemäss einem der Ansprüche 4 bis 6, **dadurch gekennzeichnet, dass** das Quarternisierungsmittel oder die Quarternisierungsmittel (III) über einen Zeitraum von 0,5 bis 2 Stunden in die Lösung der Verbindung (III) eingebracht wird.

8. Verfahren gemäss einem der Ansprüche 4 bis 7, **dadurch gekennzeichnet, dass** die Reaktion der Verbindungen (II) und (III) nach dem Einbringen der Gesamtmenge des Quarternisierungsmittels oder der Quarternisierungsmittel im Verlauf von 10 bis 40 Stunden durchgeführt wird.

9. Verfahren gemäss einem der Ansprüche 4 bis 8, **dadurch gekennzeichnet, dass** aus der erhaltenen wässrigen Lösung jede Spur eines organischen Lösungsmittels durch Strippen unter vermindertem Druck entfernt wird.

10. Verfahren gemäss einem der Ansprüche 4 bis 9, **dadurch gekennzeichnet, dass** man eine wässrige Lösung bis zu einer Konzentration der Verbindung 1 von 65 bis 75 Gewichtsprozent aufkonzentriert.

11. Verfahren gemäss einem der Ansprüche 4 bis 10, **dadurch gekennzeichnet, dass** es in der Gegenwart von wenigstens einem Stabilisierungsmittel, ausgewählt insbesondere aus Hydrochinon, Hydrochinonmethylether und 3,5-di-tert.-butyl-4-hydroxytoluol und Mischungen dieser Stabilisierungsmittel, durchgeführt wird, wobei der Gehalt an Stabilisierungsmittel(n) insbesondere von 400 bis 2000 ppm im Verhältnis zur endgültigen, wässrigen Lösung der Verbindung (I) beträgt.

12. Verfahren gemäß einem der Ansprüche 4 bis 11, dadurch gekennzeichent, dass die Verbindung (II) durch Umsetzen einer Verbindung der Formel (IV): 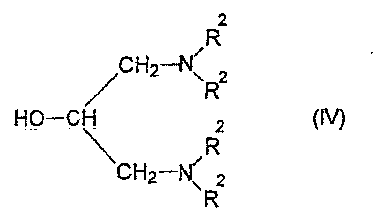 worin R² wie oben definiert ist,
mit (Meth)acrylsäureanhydrid in Gegenwart von Triethylamin, bei einem Molverhältnis von (Meth)acrylsäureanhydrid/Verbindung (IV) von 0,5 bis 2, bei einer Temperatur von 20 bis 100 °C, insbesondere von 30 bis 60 °C, im Verlauf von 2 bis 10 Stunden, in Gegenwart von wenigstens einem Stabilisierungsmittel, wie Phenothiazin, Hydrochinonmethylether, 3,5-di-tert.-butyl-4-Hydroxytoluol und Hydrochinon, und den Mischungen dieser Stabilisierungsmittel, in einer Menge von 200 bis 3000 ppm in Bezug auf eine Charge.

13. Homopolymere oder Copolymere, wenigstens Monomereinheiten der Formel (I) umfassend, wobei diese wie in einem der Ansprüche 1 bis 3 definiert sind.
